**Europäisches Patentamt**

**European Patent Office**

**Office européen des brevets**

(19)

(11) Veröffentlichungsnummer: **0 007 057**
**B1**

(12) **EUROPÄISCHE PATENTSCHRIFT**

(45) Veröffentlichungstag der Patentschrift:
26.08.81

(51) Int. Cl.³: **G 01 N 33/96,** A 61 K 35/16,
C 12 Q 1/50

(21) Anmeldenummer: **79102244.5**

(22) Anmeldetag: **03.07.79**

(54) Kontrollserum für die klinisch-chemische Analyse mit einem bestimmten Gehalt an Creatin-kinase.

(30) Priorität: **17.07.78 DE 2831390**

(43) Veröffentlichungstag der Anmeldung:
**23.01.80 Patentblatt 80/2**

(45) Bekanntmachung des Hinweises auf die Patenterteilung:
**26.08.81 Patentblatt 81/34**

(84) Benannte Vertragsstaaten:
**BE CH DE FR GB IT LU NL SE**

(56) Entgegenhaltungen:
**DE-A1-2 353 035**
**DE-A1-2 442 680**
**DE-A1-2 648 759**
**US-A-3 540 984**

(73) Patentinhaber: **BOEHRINGER MANNHEIM GMBH,**
**Sandhofer Strasse 112-132 Postfach 31 01 20,**
**D-6800 Mannheim 31-Waldhof (DE)**

(72) Erfinder: **Hundt, Dieter, Dr., Bergerstrasse 15a,**
**D-8136 Percha (DE)**
Erfinder: **Sedelmeyer, Marianne, Am Frischanger 9,**
**D-8120 Weilheim (DE)**
Erfinder: **Röschlau, Peter, Dr., Schönegertstrasse 4,**
**D-8124 Seeshaupt (DE)**
Erfinder: **Stähler, Fritz, Dr., Heimgartenstrasse 4,**
**D-8132 Tutzing (DE)**
Erfinder: **Gruber, Wolfgang, Dr., Am Oberanger 7,**
**D-8132 Tutzing-Unterzeismering (DE)**

(74) Vertreter: **Weickmann, Heinrich, Dipl.-Ing Patentanwälte**
**Dipl.Ing.H.Weickmann et al, Dipl.Phys.Dr.K.Fincke**
**Dipl.Ing.F.A.Weickmann Dipl.Chem.B.Huber,**
**Dr.-Ing.H.Liska Möhlstrasse 22, D-8000 München 86 (DE)**

Kontrollserum für die klinisch-chemische Analyse mit einem bestimmten Gehalt an Creatin-kinase.

Die Erfindung betrifft ein Kontrollserum, welches Creatin-kinase konstanter Aktivität enthält.

Die Bestimmung der Creatin-kinase (CK) spielt in der klinisch-chemischen Analyse eine wichtige Rolle. Zur Kontrolle derartiger Analysen ist es erforderlich, die Richtigkeit der Analyse ständig anhand von Kontrollseren mit bekannter Aktivität an Creatin-kinase zu kontrollieren. Derartige Kontrollseren, bei Creatin-kinase vorwiegend auf der Basis von Humanserum bzw. Rinderserumalbumin, müssen zur Überprüfung der Richtigkeit der Analysenergebnisse einer Vielzahl von Komponenten geeignet sein. Hierbei müssen sogenannte Soll-Werte in einem bestimmten Toleranzbereich wiedergefunden werden. Dieses Konzept setzt voraus, dass die Komponenten im gebrauchsfertigen Kontrollserum in einem vorgegebenen Zeitraum stabil sind, d.h., dass sich ihre Konzentration oder Aktivität in diesem Zeitraum nicht oder zumindest nicht wesentlich verändert. Die Erreichung dieser Stabilität wirft bei Enzymen als Komponenten solcher Kontrollseren beträchtliche Probleme auf.

In Kontrollseren, die üblicherweise auf der Basis von Humanserum, tierischem Serum bzw. Rinderserumalbumin aufgebaut sind, muss die CK-Aktivität durch Aufstocken erhöht werden, um eine gute Messbarkeit und gute Präzision zu gewährleisten. Unter «Aufstocken» wird hier der Zusatz von exogener CK-Aktivität, über die endogene, im Basismaterial schon vorhandene Aktivität hinaus, verstanden. Die aufgestockte CK-Aktivität aber ist in dem gebrauchsfertigen Kontrollserum, insbesondere bei Raumtemperatur, nicht ausreichend lange stabil, so dass die bei der Qualitätskontrolle erforderliche Wiederfindung des Soll-Werts oftmals problematisch ist. Über diese in einem Kontrollserum äusserst unerwünschte Eigenschaft der Creatin-kinase, in der rekonstituierten Kontrollprobe rasch an Aktivität zu verlieren, wird z.B. in Z. Klin. Chem. Klin. Biochem. 8, 212–217 (1970) berichtet. Dieser rasche Abfall der Aktivität, der bei Raumtemperatur bis zu 40% innerhalb weniger Stunden betragen kann, zwingt in der Regel dazu, nur ganz frisch rekonstituiertes Kontrollserum einzusetzen. Das ist nicht nur unpraktikabel, sondern auch unwirtschaftlich.

Der Erfindung liegt daher die Aufgabe zugrunde, diese Nachteile zu beseitigen und ein Kontrollserum mit konstanter Aktivität der Creatin-kinase zu schaffen, welches mit einem Minimum an Arbeitsaufwand, insbesondere auch in Gegenwart anderer Enzyme, angewandt werden kann, ohne deren Aktivität zu beeinflussen.

Gelöst wird diese Aufgabe erfindungsgemäss durch ein Kontrollserum auf Basis von Humanserum, Pferdeserum, Rinderserum, Rinderserumalbumin oder Humanserumalbumin mit bestimmtem Gehalt an zusätzlicher Creatin-kinase und gegebenenfalls anderen Enzymen, Substraten, Metaboliten und anderen, im Serum vorkommenden Substanzen, welches dadurch gekennzeichnet ist, dass es als zusätzliche Creatin-kinase Schweineserum enthält.

Bisher war es üblich, aus tierischem Gewebe isolierte und gereinigte Creatin-kinase zum Aufstocken von Kontrollseren zu verwenden (vgl. «Fortschritte der Klinischen Chemie, Enzyme und Hormone», Tagungsbericht Wien, 22.–24.4.1971, S. 346 ff., Verlag der Wiener Med. Akademie, 1972). Die Erfindung beruht auf der überraschenden Feststellung, dass bei Einsatz von vollständigem Schweineserum anstelle der bisher üblichen gereinigten Gewebeenzympräparate im vollständigen Kontrollserum eine überraschend hohe und konstante CK-Aktivität aufrechterhalten wird. Mit gereinigter CK aus Schweineserum lässt sich dieser Effekt nicht erzielen. Die Ursache für diese überraschende Eigenschaft des Schweineserums ist nicht bekannt. Es wird jedoch angenommen, dass das vollständige Schweineserum auf die CK eine stabilisierende Wirksamkeit ausübt, die verlorengeht, wenn man die darin enthaltene CK aufreinigt bzw. isoliert.

Bisher war es üblich, dem Kontrollserum zur Aufstockung isolierte und gereinigte CK aus tierischem Gewebe, wie z.B. Kaninchenmuskel, zuzusetzen. Das erfindungsgemäss eingesetzte Schweineserum weist an sich gute endogene CK-Aktivität auf, verglichen mit der endogenen CK-Aktivität von Humanserum. Daher reicht es im allgemeinen völlig aus, wenn auf 50 bis 150 Vol.-Teile gebrauchsfertiges Kontrollserummedium 1 Vol.-Teil Schweineserum zugesetzt wird, jedoch können auch grössere und kleinere Mengen zugesetzt werden, wie 1:20 bis 150 Vol.-Teile. Die Volumenangaben beziehen sich auf in üblicher Weise gewonnenes Serum. Schweineserum in diesen Mengen ruft keine Störungen im Kontrollserum (z.B. bei der Eiweisselektrophorese) hervor. Die Erfindung eignet sich für alle üblichen Kontrollserenmedien, wie Human-, Rinder-, Pferdeserum und Rinderserumalbuminlösung.

Kontrollseren auf Basis von Schweineserum sind bekannt («Progress in Quality Control in Clinical Chemistry», Vth International Symposium, Geneva, 1973, S. 268–276). Aufgrund ihrer hohen CK-Aktivität müssen diese jedoch vor der Verwendung stark verdünnt werden. Hierdurch wird eine gravierende Fehlerquelle geschaffen.

Als Vorbereitung für die Verwendung von Schweineserum als «zusätzliche Creatin-kinase» im erfindungsgemässen Kontrollserum genügt eine Entfernung von unlöslichen Bestandteilen, beispielsweise durch Filtration. Weitere vorbereitende Verfahrensschritte sind nicht erforderlich. Da keinerlei Aufreinigung erforderlich ist, werden auch Verfahrensschritte, wie sie bei der bisher bekannten Aufstockung mit gereinigter CK notwendig sind, nicht erforderlich.

Die folgenden Beispiele erläutern die Erfindung weiter.

Beispiel 1

100 ml Humanserum wurden mit den Enzymen GOT, GPT, LDH, GIDH, ALD, LAP, α-Amylase, SP, AP, γ-GT und Lipase versetzt. Weiterhin wurden Glucose, Creatinin, Harnstoff, Harnsäure und Bilirubin zugesetzt. Die CK-Aktivität wurde mit dem aus Kaninchenmuskel isolierten und gereinigten Enzym auf einen gewünschten Wert eingestellt. Die erhaltene Lösung wird klar filtriert und in Portionen zu 5 ml in Fläschchen abgefüllt. Nach der Lyophilisation werden die Proben bei +4°C gelagert. Die Aktivitätsänderung in der mit 5 ml bidestilliertem Wasser rekonstituierten und bei +25°C gelagerten Probe zeigt nachfolgende Tabelle.

| 2 | 4 | 6 | 24 | (Stunden) |
|---|---|---|---|---|
| -21 | -28 | -31 | -47 | (%) |

Beispiel 2

Das Verfahren von Beispiel 1 wird wiederholt, jedoch bilden 100 ml 6% Rinderserumalbumin-Lösung die Ausgangsbasis. Die CK-Aktivität wird wiederum mit dem aus Kaninchenmuskel isolierten und gereinigten Enzym eingestellt. Die Aktivitätsänderung in der rekonstituierten und bei +25°C gelagerten Probe zeigt nachfolgende Tabelle.

| 2 | 4 | 6 | 24 | (Stunden) |
|---|---|---|---|---|
| -9 | -17 | -20 | -40 | (%) |

Beispiel 3

Das Verfahren von Beispiel 1 wird wiederholt, wobei abermals 10 ml Humanserum als Ausgangsbasis dienen. Die CK-Aktivität wird durch Zusatz von Schweineserum eingestellt. Humanserum + Schweineserum = 100 + 1 (v + v). Nachfolgende Tabelle zeigt die Aktivitätsänderung in der rekonstituierten und bei +25°C gelagerten Probe.

| 2 | 4 | 6 | 24 | (Stunden) |
|---|---|---|---|---|
| -4 | -6 | -7 | -10 | (%) |

Beispiel 4

Das Verfahren von Beispiel 1 wird wiederholt, wobei jedoch 100 ml 6% Rinderserumalbumin-Lösung als Ausgangsbasis dienen. Die CK-Aktivität wird durch Zusatz von Schweineserum eingestellt. Rinderserumalbumin-Lösung + Schweineserum = 100 + 1 (v + v). Die Aktivitätsänderung in der rekonstituierten und bei +25°C gelagerten Probe zeigt nachfolgende Tabelle.

| 2 | 4 | 6 | 24 | (Stunden) |
|---|---|---|---|---|
| +1 | -2 | -3 | -3 | (%) |

Beispiel 5

Das Verfahren von Beispiel 1 wird wiederholt, wobei jedoch 100 ml Rinderserum als Ausgangsbasis dienen. Die CK-Aktivität wird durch Zusatz von Schweineserum eingestellt. Rinderserum + Schweineserum = 100 + 1 (v + v). Die Aktivitätsänderung in der rekonstituierten und bei +25°C gelagerten Probe zeigt nachfolgende Tabelle.

| 2 | 4 | 6 | 24 | Stunden) |
|---|---|---|---|---|
| -2 | -4 | -2 | -9 | (%) |

Beispiel 6

Das Verfahren von Beispiel 1 wird wiederholt, wobei jedoch 100 ml Pferdeserum als Ausgangsbasis dienen. Die CK-Aktivität wird durch Zusatz von Schweineserum eingestellt. Pferdeserum + Schweineserum = 100 + 1 (v + v). Die Aktivitätsänderung in der rekonstituierten und bei +25°C gelagerten Probe zeigt nachfolgende Tabelle.

| 2 | 4 | 6 | 24 | (Stunden) |
|---|---|---|---|---|
| -2 | -5 | -4 | -21 | (%) |

**Patentansprüche**

1. Kontrollserum auf Basis von Humanserum, Pferdeserum, Rinderserum, Rinderserumalbumin oder Humanserumalbumin mit bestimmtem Gehalt an zusätzlicher Creatin-kinase und gegebenenfalls anderen Enzymen, Substraten, Metaboliten und anderen, im Serum vorkommenden Substanzen, dadurch gekennzeichnet, dass es als zusätzliche Creatin-kinase Schweineserum enthält.

2. Kontrollserum nach Anspruch 1, dadurch gekennzeichnet, dass es 1 Vol.-Teil Schweineserum auf 50 bis 150 Vol.-Teile Kontrollserummedium enthält.

3. Kontrollserum nach Anspruch 1 oder 2 in lyophilisierter oder mit wässrigem Medium rekonstituierter Form.

**Claims**

1. Control serum based upon human serum, horse serum, bovine serum, bovine serum albumin or human serum albumin with a definite content of additional creatine kinase and possibly of other enzymes, substrates, metabolites and other substances occurring in serum, characterised in that it contains pig serum as additional creatine kinase.

2. Control serum according to claim 1, characterised in that it contains 1 part by volume of pig serum per 50 to 150 parts by volume of control serum medium.

3. Control serum according to claim 1 or 2, in lyophilised form or in a form reconstituted with an aqueous medium.

**Revendications**

1. Sérum témoin à base de sérum humain, de sérum de cheval, de sérum de bovin, d'albumine de sérum de bovin ou d'albumine de sérum humain avec une teneur déterminée en créatine-kinase additionnelle et éventuellement d'autres enzymes, substrats, métabolites et autres substances présentes dans le sérum, caractérisé en ce qu'il renferme, en tant que créatine-kinase additionnelle, du sérum de porc.

2. Sérum témoin selon la revendication 1, caractérisé en ce qu'il renferme 1 partie en volume de sérum de porc pour 50 à 150 parties en volume de milieu de sérum témoin.

3. Sérum témoin selon la revendication 1 ou 2 sous forme lyophilisée ou reconstituée avec du milieu aqueux.